(19) Europäisches Patentamt
European Patent Office
Office européen des brevets

(11) Publication number: **0 348 505 A1**

(12) # EUROPEAN PATENT APPLICATION

published in accordance with Art. 158(3) EPC

(21) Application number: **88900125.1**

(22) Date of filing: **21.12.87**

Data of the international application taken as a basis:

(86) International application number: **PCT/JP 87/01004**

(87) International publication number: **WO 89/05807 (29.06.89 89/14)**

(43) Date of publication of application: **03.01.90 Bulletin 90/1**

(84) Designated Contracting States: **DE FR GB IT**

(51) Int. Cl.⁴: **C 07 D 333/20**, C 07 D 333/28, C 07 D 333/32, A 01 N 43/10

(71) Applicant: **DAIKIN INDUSTRIES, LIMITED, Umeda Center Building, 4-12, 2-chome Nakasakinishi, Kita-ku, Osaka-shi, Osaka 530 (JP)**

(72) Inventor: **ITAMI, Yasuo, 5-7-16, Hishiyanishi, Higashiosaka-shi Osaka 577 (JP)**
Inventor: **HARADA, Takeshi, 2-21-21, Hitotsuya, Settsu-shi Osaka 564 (JP)**
Inventor: **SEKI, Eiji, 2-21-21, Hitotsuya, Settsu-shi Osaka 564 (JP)**
Inventor: **KITAHARA, Katsuhiko, 24, Higashihirai-cho Kitashirakawa, Sakyo-ku Kyoto-shi Kyoto 606 (JP)**

(74) Representative: **Hansen, Bernd, Dr.rer.nat., Hoffmann, Eitle & Partner Patentanwälte Arabellastrasse 4 Postfach 81 04 20, D-8000 München 81 (DE)**

(54) **NOVEL N-(1-THIENYLETHYL)AMINE DERIVATIVES AND HERBICIDES CONTAINING THE SAME AS EFFECTIVE INGREDIENT.**

(57) N-(1-thienylethyl)amine derivatives represented by formula (I) are disclosed, wherein Z represents a halogen atom, a lower alkyl group or a lower alkoxy group, n represents 0, 1 or 2, and R represents (a) (wherein X and Y, which may be the same or different, each represents hydrogen, a straight-chain or branched lower alkyl group optionally being bound to each other to form a ring, a straight-chain or branched lower haloalkyl group, a straight-chain or branched lower alkoxy group, a straight-chain or branched lower alkoxymethyl group or a halogen, provided that both of X and Y do not represent hydrogen atoms at the same time) or (b), wherein Z and n are the same as defined above. These derivatives show a strong herbicidal effect on annual weed growing in a paddy field and weed growing in an upland field.

EP 0 348 505 A1

0 348 505

SPECIFICATION

# NOVEL N-[1-(THIENYL)ETHYL]AMINE DERIVATIVE AND HERBICIDE CONTAINING THE DERIVATIVE AS ACTIVE INGREDIENT

[FIELD OF THE INVENTION]

The present invention relates to a novel N-[1-(thienyl)ethyl]amine derivative and to a herbicide containing said derivative as an active ingredient. The present compound shows high herbicidal activity and is extraordinarily effective against lowland annual weeds such as Echinochloa oryzicola Vasing., Cyperus difformis L., Monochoria vaginalis Presl, Lindernia procumbens Philcox, Rotala indica Koehne and Elatine triandra Schk. and against upland weeds such as Echinochloa frumentacea Link, Digitaria adscendens Henr., Abutilon avicennae Gaertn., Amaranthus viridis L. and Bidens biternata Merr. et Sherff.

[BACKGROUND OF THE INVENTION]

Farmaco. Ed. Sci., 34, 338 - 45 (1979) describes that a certain N-benzylbenzamide compound is herbicidally active. However, the herbicidal activity of said compound is low in upland soil treatment, although it may be more or less effective. The compound is hardly herbicidally active in irrigated-soil treatment and upland foliage treatment.

Although N-[1-(2-thienyl)ethyl]-benzamide, which is an analogous compound to the present compound, is known from

Z. Chem., 9, 448 - 9 (1969), the herbicidal activity thereof has not been reported. An examination by the present inventors shows that said compound has unsatisfactory herbicidal activity in upland foliage- and soil-treatment and is hardly effective in irrigated-soil treatment. N-[1-(2-Thienyl)ethyl]-3,5-dinitrobenzamide is also a known compound described in J. Chromatogr., 192, 143 - 158 (1980), herbicidal activity of which has not been known. According to another examination by the present inventors, this compound has almost no herbicidal activity.

[DESCRIPTION OF THE INVENTION]

According to one aspect of the present invention, provided is an N-[1-(thienyl)ethyl]amine derivative of the formula:

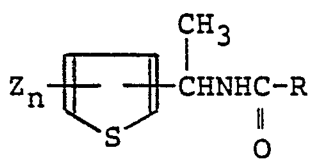

(I)

wherein

Z is halogen, a lower alkyl group or a lower alkoxy group,

n is 0, 1 or 2,

R is 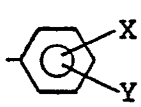

(wherein X and Y, which are the same or different, represent hydrogen, straight or branched lower alkyl groups (X and Y may form a ring), straight or branched lower haloalkyl groups, straight or branched lower alkoxy groups, straight

or branched lower alkoxymethyl groups or halogen, both of X and Y being not hydrogen), or

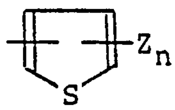

(wherein Z and n are the same as defined above).

According to another aspect of the present invention, a herbicide containing the N-[1-(thienyl)ethyl]-amine derivative of the formula (I) as an active ingredient is provided.

Typical examples of the present compounds are as follows. However, the invention is not restricted by the following compounds.

Compound No. Nomenclature of compound (Structural formula)

1 N-[1-(2-thienyl)ethyl]-4-fluorobenzamide

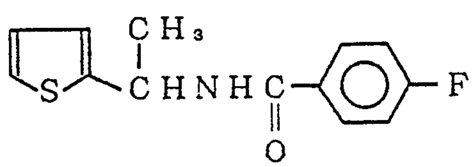

2 N-[1-(2-thienyl)ethyl]-4-chlorobenzamide

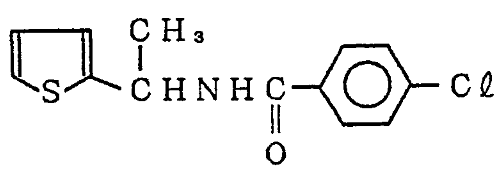

3 N-[1-(2-thienyl)ethyl]-4-bromobenzamide

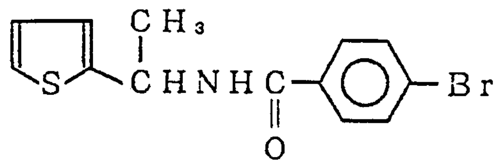

4 N-[1-(2-thienyl)ethyl]-3-chlorobenzamide

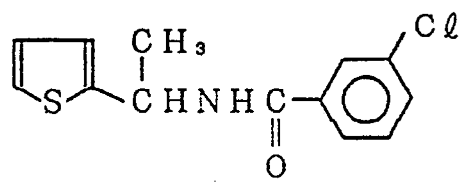

5 N-[1-(2-thienyl)ethyl]-3,4-dichlorobenzamide

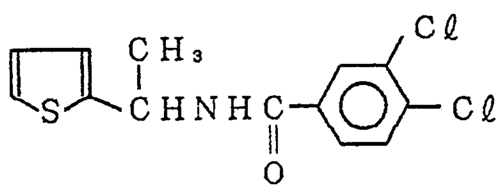

6 N-[1-(2-thienyl)ethyl]-2,4-dichlorobenzamide

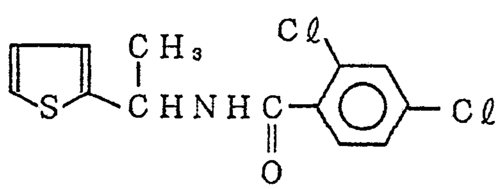

7 N-[1-(2-thienyl)ethyl]-3-methoxybenzamide

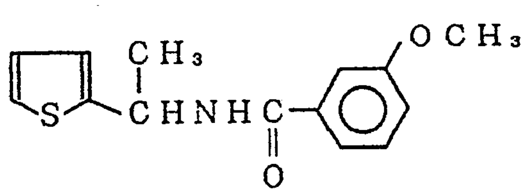

8 N-[1-(2-thienyl)ethyl]-4-methoxybenzamide

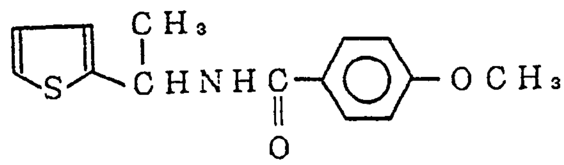

9 N-[1-(2-thienyl)ethyl]-4-isopropoxybenzamide

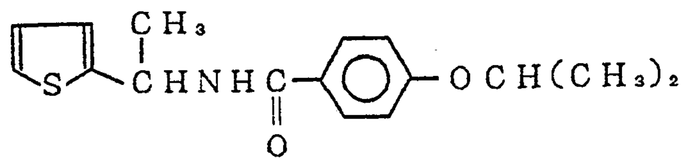

10 N-[1-(2-thienyl)ethyl]-3,4-dimethoxybenzamide

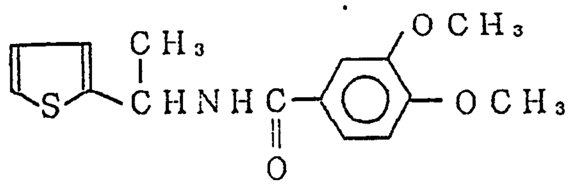

11 N-[1-(2-thienyl)ethyl]-2-methylbenzamide

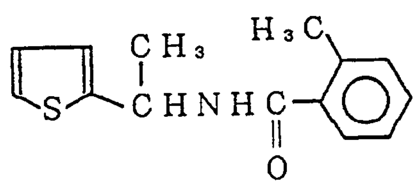

12 N-[1-(2-thienyl)ethyl]-3-methylbenzamide

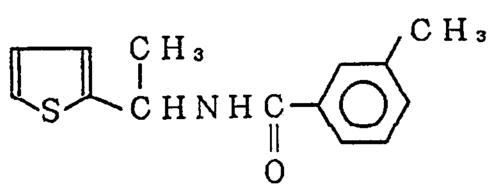

13 N-[1-(2-thienyl)ethyl]-4-methylbenzamide

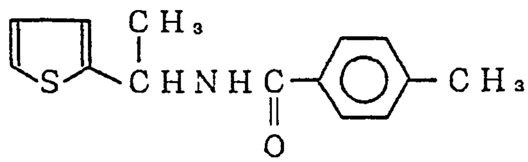

14 N-[1-(2-thienyl)ethyl]-4-isopropylbenzamide

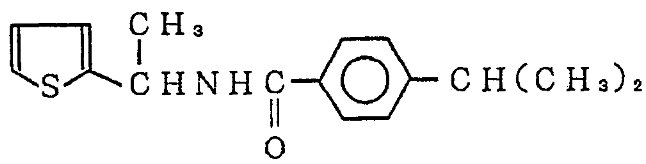

15 N-[1-(2-thienyl)ethyl]-4-t-butylbenzamide

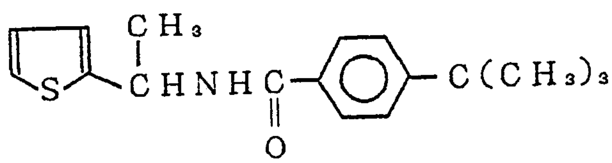

16 N-[1-(2-thienyl)ethyl]-5,6,7,8-tetrahydro-β-naphtamide

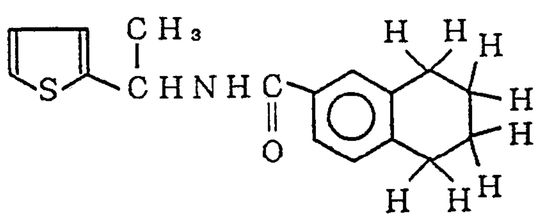

17 N-[1-(2-thienyl)ethyl]-3,4-dimethylbenzamide

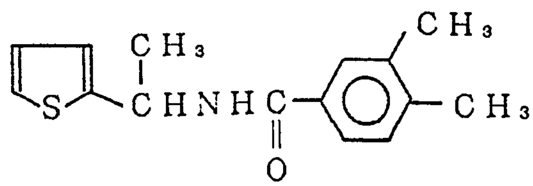

18 N-[1-(2-thienyl)ethyl]-4-(bromomethyl)benzamide

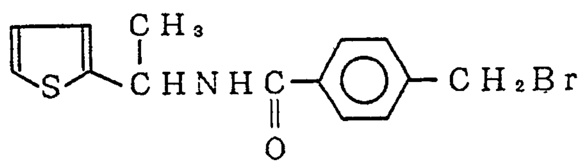

19 N-[1-(2-thienyl)ethyl]-3-methyl-4-bromobenzamide

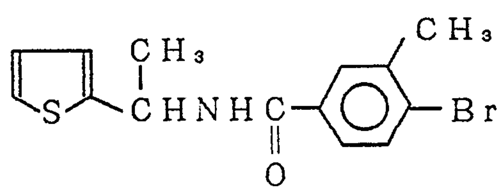

20 N-[1-(2-thienyl)ethyl]-4-trifluoromethylbenzamide

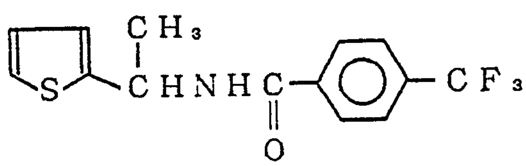

21 N-[1-(2-thienyl)ethyl]-3-trifluoromethylbenzamide

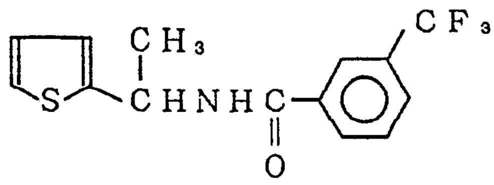

22 N-[1-(2-thienyl)ethyl]-4-(methoxymethyl)benzamide

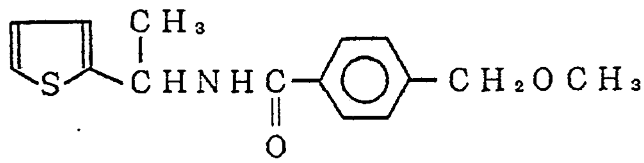

23 N-[1-(2-thienyl)ethyl]-2-thiophenecarboxylic acid amide

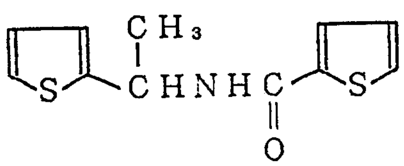

24 N-[1-(2-thienyl)ethyl]-5-methyl-2-thiophenecarboxylic acid amide

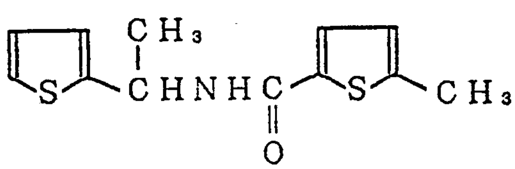

25 N-[1-(5-chloro-2-thienyl)ethyl]-4-chlorobenzamide

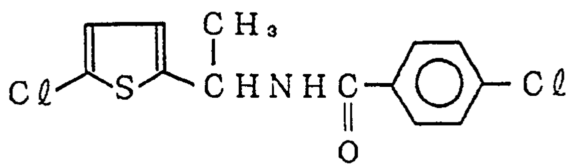

26 N-[1-(5-chloro-2-thienyl)ethyl]-4-methylbenzamide

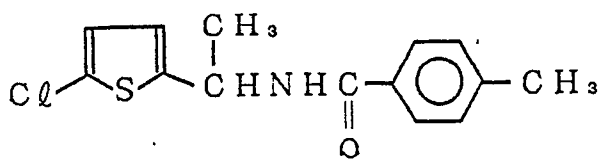

27 N-[1-(5-chloro-2-thienyl)ethyl]-4-isopropylbenzamide

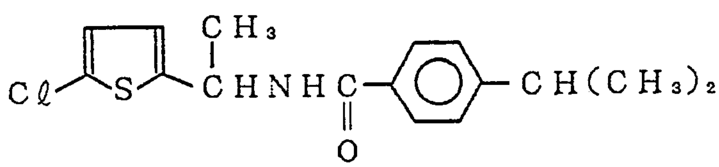

The present compound can be prepared, for example, according to the following reaction scheme:

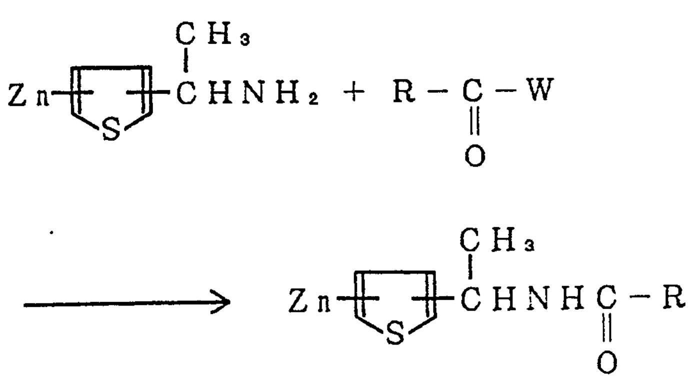

wherein R, Z and n are the same as defined above, and W is halogen.

In this reaction, a solvent such as benzene, diethyl ether, chloroform, acetone, tetrahydrofuran or dioxane and an acid-scavenger such as triethylamine, pyridine, N,N-dimethylaniline or N-methylmorphrine are usually used. The reaction temperature is preferably in the range of from - 20 to 50 °C.

The present compound can be used as a herbicide as such or in a preparation form such as granule, dust, water-dispersible powder, emulsion or aqueous suspension.

The granule contains about 0.1 to 30 % by weight of the present compound as the active ingredient and can be prepared by coating a granular inactive substrate, for example grains of sand having a particle size of about 0.2 to 2 mm, with the present compound, with adding a binder, if necessary. The dust contains about 0.1 to 20 % by weight of the present compound as the active ingredient and can be prepared by size-reducing and mixing the present compound with a solid diluent such as kieselguhr, talc, mica, acid clay or quartz powder, and, if necessary, an additive such as an oil absorbent, a lubricant or a stabilizer. The water-dispersible powder contains about 10 to 80 % by weight of the present compound as the active ingredient and can be prepared by throughly size-reducing and mixing the present compound with a solid diluent such as clay mineral and a

surfactant such as a nonionic surfactant. The emulsion contains about 5 to 80 % by weight of the present compound as the active ingredient and can be prepared by mixing the present compound with an organic solvent and an emulsifier such as an anionic or nonionic surfactant. The aqueous suspension contains 5 to 70 % by weight of the present compound as the active ingredient and can be prepared by mixing the present compound in a size-reduced form with water and a wetting agent or a dispersant, and so on.

So as to increase the herbicidal activity, the present compound can be used in combination with other herbicide, for example a diphenyl ether herbicide such as 2,4-dichlorophenyl 4'-nitrophenyl ether and 2,4,6-trichlorophenyl 4'-nitrophenyl ether; a triazine herbicide such as 2-chloro-4,6-bisethylamino-1,3,5-triazine and 2-methylthio-4,6-bisethylamino-1,3,5-triazine; a urea herbicide such as 3-(3,4-dichlorophenyl)-1,1-dimethylurea; a carbamate herbicide such as methyl N-(3,4-dichlorophenyl)carbamate; a thiocarbamate herbicide such as S-(4-chlorobenzyl)-N,N-diethylthiolcarbamate or S-ethyl-N,N-hexamethylenethiocarbamate; an acid amide herbicide such as 3,4-dichloropropionanilide or 2-chloro-2',6'-diethyl-N-(butoxymethyl)-acetanilide; a pyridinium herbicide such as 1,1'-dimethyl-4,4-bispyridinium chloride; a phosphorus-containing herbicide such as N,N-bis(phosphonomethyl)-glycine, S-(2-methyl-1-piperidylcarbonylmethyl)O,O-di-n-propyldithiophos-

phate; a pyrazolate herbicide such as 4-(2,4-dichlorobenzoyl)-1,3-dimethyl-pyrazol-5-yl-p-toluenesulfonate; and a toluidine herbicide such as α,α,α-trifluoro-2,6-dinitro-N,N-dipropyl-p-toluidine.

It is also possible, if necessary, to use the present compound in combination with an insecticide, a nematicide, a bactericide, a growth-regulating agent of plants and a fertilizer.

[EFFECTS OF THE INVENTION]

The present compound shows extraordinary herbicidal activity in irrigated-soil treatment. It is excellently effective particularly against annual weeds such as Echinochloa oryzicola Vasing., Cyperus difformis L., Monochoria vaginalis Presl, Lindernia procumbens Philcox, Rotala indica Koehne and Elatine triandra Schk. as well as against Spirodela polyrhiza Schleid. In addition, the present compound is highly effective against algae and, therefore, is useful to prevent the peeling of soil surface caused by the algae. The compound has another excellent characteristic that it never injure rice even immediately after inoculation or transplantation.

Since the compound has a high algaecidal activity, it can be used to prevent the emergence of algae in a lowland field as well as in a reservoir, an irrigation canal and industrial circulating water.

The present compound also shows a high herbicidal action on upland weeds such as Echinochloa oryzicola Vasing., Cyperus difformis L., Monochoria vaginalis Presl, Lindernia procumbens Philcox, Rotala indica Koehne and Elatine triandra Schk. The compound can be effectively applied by any means at any time, for example during soil treatment before the germination of weed seeds and during foliage treatment against weeds in an early stage of growth.

In addition, the compound is effective as a bactericide.

[EXAMPLE]

The invention is further illustrated in detail by the following Examples and Examination Examples.

Example 1

Preparation of N-[1-(2-thienyl)ethyl]-4-fluorobenzamide (compound 1)

Into a 100 ml three-necked flask containing 50 ml of anhydrous ether, 1.27 g (0.01 mole) of 1-(2-thienyl)-ethylamine and 1.2 g of triethylamine, 1.58 g (0.01 mole) of 4-fluorobenzoyl chloride was dropwise added with stirring and cooling by ice. After the addition was completed, the mixture was stirred for one hour at a room temperature. After the reaction, the reaction solution was washed with water. Then, the organic layer was dried on anhydrous sodium sulfate. The solvent was distilled off under a reduced pressure to obtain a crude crystalline product. The

crude product was subjected to recrystallization from ethyl acetate/n-hexane to obtain 2.05 g of the desired compound, N-[1-(2-thienyl)ethyl]-4-fluorobenzamide. Melting point of the compound was 85 °C.

In the same way, the above-mentioned compounds were prepared and melting points thereof were determined. The melting points determined are shown in Table 1.

Example 2

60 Parts by weight of talc, 15 parts by weight of Monogen powder (which is a surfactant produced by Dai-ichi Kogyo Seiyaku Co., Ltd.) and 25 parts by weight of each test compound listed in Table 2 were throughly size-reduced and mixed to prepare a water-dispersible powder.

Examination Example 1

Irrigated-soil treatment

A 1/10,000 are Wagner pot was filled with lowland soil. The surface of the soil was sown with seeds of each test weed. Seedlings of rice in 2 to 3 leaf stage were transplanted at a depth of 0 cm and 2 cm in the soil. Then, the pot was irrigated. After a water level of 3 cm was maintained for 3 days, a liquid prepared by diluting the each water-dispersible powder obtained in Example 2 was dropwise added into the pot. Then, the pot was placed in a green house. After 30 days from the treatment, damage of the plants was observed with regard to the number of germinated seeds, plant length, condition of the plants, and

so on. Herbicidal and algaecidal effects and crop injury were evaluated according to the observation and expressed by a number of 0 to 5 according to the following criteria. The results are shown in Table 2.

Herbicidal and algaecidal effects

0: no effect

1: slight effect

2: moderate effect

3: high effect

4: excellent effect

5: withering off

Crop injury

0: no injury

1: slight injury

2: moderate injury

3: considerable injury

4: excessive injury

5: extreme injury (withering)

Examination Example 2

Upland soil treatment before the emergence of weeds

Upland soil filled in a 1/88,500 x 10 are plastic vessel was seeded with each test plant and covered with further amount of the soil. Then, the water-dispersible powder of the each test compound prepared in Example 2 was diluted so as to apply 100 l of the powder per 10 are and

dropwise applied on the soil. Then, the pot was placed in a green house. After 21 days from the treatment, damage of the plant was observed with regard to the number of germinated seeds, plant length, condition of the plant, and so on. Herbicidal effect was evaluated according to the observation and expressed by a number of 0 to 5 according to the same criteria as in Examination Example 1. The results are shown in Table 3.

Examination Example 3

Foliage treatment against growing upland weeds

Upland soil filled in a 1/88,500 x 10 are plastic vessel was seeded with each test plant. The plant was then grown in a green house to 1 to 2 leaf stage. The water-dispersible powder of the each test compound prepared in Example 2 was diluted so as to apply 100 l of the powder per 10 are and applied onto the plant with a small atomizer. Then, the pot was placed in a green house. After 21 days from the treatment, damage of the plant was observed with regard to the number of germinated seeds, plant length, condition of the plant, and so on. Herbicidal effect was evaluated according to the observation and expressed by a number of 0 to 5 according to the same criteria as in Examination Example 1. The results are shown in Table 4.

Table 1

| Compound No. | m.p. (°C) | Compound No. | m.p. (°C) |
|---|---|---|---|
| (1) | 85 | (15) | 112 |
| (2) | 104 | (16) | 99 - 100 |
| (3) | 116 | (17) | 131 |
| (4) | 72 | (18) | 121.5 |
| (5) | 133 | (19) | 169 |
| (6) | 78 | (20) | 109 |
| (7) | 92 - 3 | (21) | 116 |
| (8) | 114 | (22) | 66.5 |
| (9) | 98 - 9 | (23) | 95 - 6 |
| (10) | 127 | (24) | 123.5 |
| (11) | 84 | (25) | 122 |
| (12) | 62 | (26) | 112 |
| (13) | 115 - 6 | (27) | 98 - 9 |
| (14) | 100 | | |

Table 2

| Test compound | Amount of applied test compound (g/a) | Herbicidal effect | | | | Algaecidal effect | Rice injury | |
|---|---|---|---|---|---|---|---|---|
| | | E* | C* | M* | The other broadleaf weeds 4) | | 0 cm in depth | 2 cm in depth |
| 1 | 100<br>50 | 5<br>5 | 5<br>5 | 5<br>5 | 5<br>5 | 5<br>5 | 0<br>0 | 0<br>0 |
| 3 | 100<br>50 | 5<br>5 | 5<br>5 | 5<br>5 | 5<br>5 | 5<br>5 | 0<br>0 | 0<br>0 |
| 5 | 100<br>50 | 5<br>5 | 5<br>5 | 5<br>5 | 5<br>5 | 5<br>5 | 0<br>0 | 0<br>0 |
| 6 | 100<br>50 | 5<br>5 | 5<br>5 | 5<br>5 | 5<br>5 | 5<br>5 | 0<br>0 | 0<br>0 |
| 8 | 100<br>50 | 5<br>5 | 5<br>4 | 5<br>4 | 5<br>5 | 5<br>5 | 1<br>0 | 0<br>0 |
| 9 | 100<br>50 | 5<br>4 | 5<br>5 | 5<br>5 | 5<br>5 | 5<br>5 | 0<br>0 | 0<br>0 |
| 14 | 100<br>50 | 5<br>5 | 5<br>5 | 5<br>5 | 5<br>5 | 5<br>5 | 0<br>0 | 0<br>0 |
| 15 | 100<br>50 | 5<br>4 | 5<br>5 | 4<br>4 | 5<br>5 | 5<br>5 | 0<br>0 | 0<br>0 |
| 16 | 100<br>50 | 5<br>5 | 5<br>5 | 5<br>5 | 5<br>5 | 5<br>5 | 0<br>0 | 0<br>0 |
| 17 | 100<br>50 | 5<br>5 | 5<br>5 | 5<br>5 | 5<br>5 | 5<br>5 | 2<br>0 | 0<br>0 |
| 18 | 100<br>50 | 5<br>4 | 5<br>5 | 5<br>5 | 5<br>5 | 5<br>5 | 2<br>0 | 0<br>0 |
| 20 | 100<br>50 | 5<br>5 | 5<br>5 | 5<br>5 | 5<br>5 | 5<br>5 | 0<br>0 | 0<br>0 |
| 21 | 100<br>50 | 5<br>5 | 5<br>5 | 5<br>5 | 5<br>5 | 5<br>5 | 1<br>0 | 0<br>0 |
| 22 | 100<br>50 | 5<br>4 | 5<br>5 | 5<br>4 | 5<br>5 | 5<br>5 | 0<br>0 | 0<br>0 |
| 24 | 100<br>50 | 5<br>5 | 5<br>5 | 5<br>5 | 5<br>5 | 5<br>5 | 2<br>1 | 0<br>0 |

Table 2 (continued)

| Test compound | Amount of applied test compound (g/a) | Herbicidal effect | | | | Algaecidal effect | Rice injury | |
|---|---|---|---|---|---|---|---|---|
| | | E* | C* | M* | The other broadleaf weeds[4)] | | 0 cm in depth | 2 cm in depth |
| Comparative compound (1)[1)] | 100<br>50 | 1<br>0 | 2<br>1 | 1<br>0 | 2<br>0 | 2<br>1 | 3<br>2 | 1<br>0 |
| Comparative compound (2)[2)] | 100<br>50 | 1<br>0 | 3<br>2 | 3<br>1 | 3<br>2 | 2<br>1 | 4<br>1 | 2<br>1 |
| Comparative compound (3)[3)] | 100<br>50 | 3<br>2 | 0<br>0 | 0<br>0 | 0<br>0 | 0<br>0 | 3<br>3 | 1<br>0 |

Note:

1) Comparative compound (1):
N-(α-methylbenzyl)benzamide
[which is a compound described in Farmaco. Ed. Sci., 34, 338 - 45 (1979)]

2) Comparative compound (2):
N-[1-(2-thienyl)ethyl]benzamide
[which is a compound described in Z. Chem. 9, 448 - 9 (1969)]

3) Comparative compound (3):
N-[1-(2-thienyl)ethyl]-3,5-dinitrobenzamide
[which is a compound described in J. Chromatogr., 192, 143 - 158 (1980)]

4) The other broadleaf weeds:
Rotala indica Koehne, Lindernia procumbens Philcox, Elatine triandra Schk. etc.

E*: Echinochloa oryzicola Vasing.

C*: Cyperus difformis L.

M*: Monochoria vaginalis Presl

Table 3

| Test compound | Amount of applied test compound (g/a) | Herbicidal effect | | | | |
|---|---|---|---|---|---|---|
| | | Ef* | Da* | B* | Ms* | Aa* |
| 1 | 50<br>25 | 5<br>5 | 5<br>5 | 5<br>5 | 5<br>5 | 5<br>5 |
| 2 | 50<br>25 | 5<br>4 | 5<br>5 | 5<br>5 | 5<br>5 | 5<br>5 |
| 7 | 50<br>25 | 3<br>2 | 5<br>3 | 5<br>5 | 5<br>5 | 4<br>3 |
| 8 | 50<br>25 | 3<br>2 | 3<br>2 | 5<br>5 | 5<br>5 | 5<br>5 |
| 12 | 50<br>25 | 5<br>5 | 5<br>5 | 5<br>5 | 5<br>5 | 5<br>5 |
| 13 | 50<br>25 | 2<br>2 | 5<br>5 | 5<br>5 | 5<br>5 | 5<br>5 |
| 23 | 50<br>25 | 5<br>5 | 5<br>5 | 5<br>5 | 5<br>5 | 5<br>5 |
| Comparative compound (1)[1)] | 50<br>25 | 0<br>0 | 0<br>0 | 3<br>2 | 4<br>1 | 1<br>1 |
| Comparative compound (2)[2)] | 50<br>25 | 4<br>3 | 2<br>0 | 1<br>0 | 4<br>3 | 3<br>2 |
| Comparative compound (3)[3)] | 50<br>25 | 0<br>0 | 0<br>0 | 0<br>0 | 0<br>0 | 0<br>0 |

Note:

1) Comparative compound (1): The same as Note 1) in Table 2

2) Comparative compound (2): The same as Note 2) in Table 2

3) Comparative compound (3): The same as Note 3) in Table 2

Ef*: Echinochloa frumentacea Link

Da*: Digitaria adscendens Henr.

B*: Brassica L. var. botrys L.

Ms*: Medicago sativa L.

Aa*: Abutilon avicennae Gaertn.

Table 4

| Test compound | Amount of applied test compound (g/a) | Herbicidal effect | | | | |
|---|---|---|---|---|---|---|
| | | Ef* | Da* | Rs* | Ms* | Aa* |
| 1 | 50<br>25 | 5<br>5 | 5<br>5 | 5<br>5 | 5<br>5 | 5<br>5 |
| 2 | 50<br>25 | 5<br>5 | 5<br>5 | 5<br>5 | 5<br>5 | 5<br>5 |
| 7 | 50<br>25 | 5<br>5 | 5<br>5 | 5<br>5 | 5<br>5 | 5<br>5 |
| 8 | 50<br>25 | 5<br>5 | 5<br>5 | 5<br>5 | 5<br>4 | 5<br>5 |
| 12 | 50<br>25 | 5<br>3 | 5<br>2 | 5<br>2 | 5<br>5 | 5<br>5 |
| 13 | 50<br>25 | 5<br>5 | 5<br>2 | 5<br>2 | 5<br>2 | 5<br>5 |
| 14 | 50<br>25 | 5<br>5 | 5<br>5 | 5<br>5 | 5<br>4 | 5<br>5 |
| 23 | 50<br>25 | 5<br>5 | 5<br>5 | 5<br>4 | 5<br>5 | 5<br>5 |
| Comparative compound (1)[1)] | 50<br>25 | 0<br>0 | 0<br>0 | 2<br>1 | 1<br>0 | 0<br>0 |
| Comparative compound (2)[2)] | 50<br>25 | 3<br>2 | 4<br>2 | 4<br>3 | 3<br>2 | 4<br>3 |
| Comparative compound (3)[3)] | 50<br>25 | 0<br>0 | 0<br>0 | 0<br>0 | 0<br>0 | 0<br>0 |

Note:
1) Comparative compound (1): The same as Note 1) in Table 2
2) Comparative compound (2): The same as Note 2) in Table 2
3) Comparative compound (3): The same as Note 3) in Table 2

Ef*: Echinochloa frumentacea Link
Da*: Digitaria adscendens Henr.
Rs*: Raphanus sativus L. var. acanthiformis Makino
Ms*: Medicago sativa L.
Aa*: Abutilon avicennae Gaertn.

What is claimed is:

1. An N-[1-(thienyl)ethyl]amine derivative of the formula:

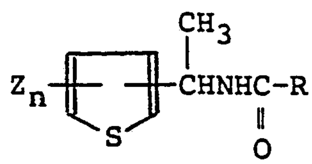

(I)

wherein

Z is halogen, a lower alkyl group or a lower alkoxy group,

n is 0, 1 or 2,

R is 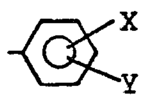

(wherein X and Y, which are the same or different, represent hydrogen, straight or branched lower alkyl groups (X and Y may form a ring), straight or branched lower haloalkyl groups, straight or branched lower alkoxy groups, straight or branched lower alkoxymethyl groups or halogen, both of X and Y being not hydrogen), or

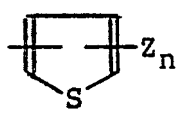

(wherein Z and n are the same as defined above).

2. A herbicide containing an N-[1-(thienyl)ethyl]-amine derivative of the formula:

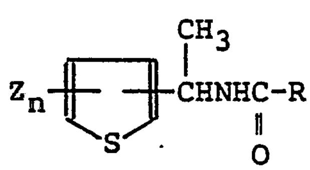

(I)

wherein

Z is halogen, a lower alkyl group or a lower alkoxy group,

n is 0, 1 or 2,

R is 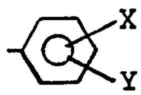

(wherein X and Y, which are the same or different, represent hydrogen, straight or branched lower alkyl groups (X and Y may form a ring), straight or branched lower haloalkyl groups, straight or branched lower alkoxy groups, straight or branched lower alkoxymethyl groups or halogen, both of X and Y being not hydrogen), or

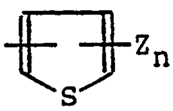

(wherein Z and n are the same as defined above)

as an active ingredient.

# INTERNATIONAL SEARCH REPORT

International Application No PCT/JP87/01004

**I. CLASSIFICATION OF SUBJECT MATTER** (if several classification symbols apply, indicate all) [3]

According to International Patent Classification (IPC) or to both National Classification and IPC

Int.Cl[4] C07D333/20, C07D333/28, C07D333/32, A01N43/10

**II. FIELDS SEARCHED**

Minimum Documentation Searched [4]

| Classification System [5] | Classification Symbols |
|---|---|
| IPC | C07D333/20, 333/28, 333/32, A01N43/10 |

Documentation Searched other than Minimum Documentation to the Extent that such Documents are Included in the Fields Searched [6]

**III. DOCUMENTS CONSIDERED TO BE RELEVANT** [14]

| Category * | Citation of Document, [16] with indication, where appropriate, of the relevant passages [17] | Relevant to Claim No. [18] |
|---|---|---|
| A | Z. Chem., Vol. 9, No. 12, (1969), Cervinka, Otakar. et al [Asymmetrische Reaktionen; 1) Die absolute Konfiguration einiger Alkohole und Amine mit Furan-und Thiophenring im Molekiil] P.448-449 | 1 |
| A | Zh. Obshch. Khim., Vol. 32, (1962) A. P. Kogerman. et al [Synthesis of some thienyl and thenyl amides of 2, 3-hydroxy-naphthoic acid and salicylic acid] P.981-983 & C. A. 58:2421h | 1 |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance: the claimed invention cannot be considered novel or cannot be considered to involve an inventive step

"Y" document of particular relevance: the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search [2] | Date of Mailing of this International Search Report [2] |
|---|---|
| February 9, 1988 (09. 02. 88) | February 22, 1988 (22.02.88) |
| **International Searching Authority** [1] | **Signature of Authorized Officer** [20] |
| Japanese Patent Office | |